# EUROPÄISCHE PATENTANMELDUNG

(11) **EP 1 525 862 A2**
(43) Veröffentlichungstag der Anmeldung: **27.04.2005**
(21) Anmeldenummer: 04024984.9
(22) Anmeldetag: 20.10.2004
(51) Int. Cl.: A61F 2/38

(54) **Tibiaplateaukomponente für eine Knieendoprothese.**

(30) Priorität: 21.10.2003 DE 10348835; 31.12.2003 DE 10361780
(71) Anmelder: Königsee Implantate und Instrumente zur Ostheosynthese GmbH, 07426 Königsee-Aschau (DE)
(72) Erfinder: Orschler, Erich, 07426 Unterschöbling (DE); Orschler, Frank, 07426 Unterschöbling (DE); Finger, Ulrich, Dr. rer. nat, 07318 Saalfeld (DE); Müller, Ulrich, Dr. med, 61231 Bad Nauheim (DE); Genrich, Heiner, 14715 Milow (DE); Kelch, Michael, 14712 Rathenow (DE)
(74) Vertreter: Kruspig, Volkmar, Dipl.-Ing.

(57) **Zusammenfassung**

Die Erfindung betrifft eine Tibiaplateaukomponente für eine Knieendoprothese, umfassend eine Tibiagleitkomponente (10), die über die Tibiagleitfläche (30) mit einer Femurkomponente (20) in gleitendem Kontakt steht und eine in der Tibia verankerte, die Tibiagleitkomponente tragende Tibiabasiskomponente (40), wobei die Tibiagleitfläche (30) mindestens entlang eines Sagittalebenenschnittes die Form eines Zylindermantelabschnittes aufweist. Der Gleitflächenradius (RG) des Zylindermantelabschnittes ist dabei gleich oder größer einem größten Femurradius (RF) der Femurkomponente (20). Weiterhin weist eine Kopplungsfläche (50) zwischen der Tibiagleitkomponente (10) und der Tibiabasiskomponente (40), die die Form eines Zylindermantelabschnittes hat einen Kopplungsflächenradius (RK) auf, dessen Größe mindestens gleich der Größe des um den Betrag der mittleren Dicke (D) der Tibiagleitkomponente erhöhten Gleitflächenradius (RG) ist.

## Beschreibung

Die Erfindung betrifft eine Tibiaplateaukomponente für eine Knieendoprothese nach dem Oberbegriff des Anspruchs 1.

Für den Fall, dass Gleitflächen der Kniegelenke eines Patienten abgenutzt sind und endoprothetisch ersetzt werden müssen, werden vorzugsweise Knie-Endoprothesen angewendet, die sich aus einer oder zwei Femurkomponenten, der Tibiabasiskomponente und den Tibiaplateau-Gleitkomponenten zusammensetzen. Hierbei werden die femuralen und tibialen Gleitflächen durch kufenartige Metallbauteile ersetzt. Die Tibiaplateaukomponente besteht aus einer in der Tibia verankerten Tibiabasiskomponente und mindestens einer und allgemeinen zwei auf diese Basiskomponente aufgeschobenen endoprothetischen Tibiagleitflächen. Die Tibiabasiskomponente besteht vorzugsweise aus einer gewebeverträglichen in den Knochen verankerten Metallkomponente, während die Tibiagleitkomponente aus einem Kunststoffmaterial ausgeführt ist und in der Tibiabasiskomponente verankert wird.

Derartige endoprothetische Elemente werden beispielsweise in der deutschen Patentschrift DE 40 11 216 C1 beschrieben. Diese offenbart insbesondere eine in den Tibiaknochen fixierbare Grundplatte mit einer planen Auflagefläche für ein lösbares verrastbares Oberteil, in dem die Gleitbahnen angeordnet sind.

Eine weitere Ausführungsform einer derartigen Tibiaplateaukomponente ist in der DE 31 36 636 A1 offenbart. In dieser Druckschrift wird ein Tibiaplateau für eine Kniegelenk-Endoprothese beschrieben, die ein als Plateau-Gleitfläche dienendes Kunststoffteil und eine Unterlagplatte aus hartem Material, insbesondere Metall aufweist. Eine Verbindung zwischen dem Kunststoffteil und der Unterlagplatte wird durch einen an der Unterlagplatte vorgesehenen Kragen mit hinterschnittener Verbindungsfläche sowie eine damit zusammenwirkende Stufe am Plateau mit einer Verbindungsfläche durch eine Rastwirkung bewirkt, die das Kunststoffteil und die Unterlagplatte aneinander halten. Die Rasteinrichtung wird von einem Zapfen und einer Bohrung gebildet.

Eine weitere Ausführungsform wird in der DE 35 35 112 A1 gezeigt. Die dort beschriebene Tibiaplateaukomponente wird in ihrer Gleitflächenkomponente durch eine Kunststoffplatte gebildet, die mit einem unteren Kunststoffkörper zusammenwirkt. Diese rastet in einen Innensechskant einer in den Knochen eingebrachten und den unteren Kunststoffkörper befestigenden Schraube, bzw. mit einer Vielzahl von Einzelzapfen, insbesondere Schnappzapfen in Perforationen des unteren Kunststoffkörpers ein. Bei dieser vorbekannten Ausführungsform kann bei intaktem Bandapparat eine Bohrung für eine Kunststoffhülse in die Tibia eingebracht werden und der untere Kunststoffkörper mit einer in die Bohrung eingreifenden Befestigungsschraube in der Tibia arretiert werden, wobei der Kunststoffkörper seitlich in das Kniegelenk eingeführt wird. Die Gleitflächenkomponente wird nachfolgend ebenfalls seitlich eingeschoben und auf dem Kunststoffkörper rastend fixiert. Diese Ausführungsform ermöglicht eine flache Ausführung der Tibia-Komponenten und ein Erhalten des Bandapparates, so dass eine Trennung dieses anatomischen Gelenkbestandteils vermieden werden kann.

Bei allen genannten Ausführungsformen ist jedoch eine gewisse Dehnung des Bandapparates unvermeidlich. Diese resultiert daraus, dass die Tibiagleitkomponente spätestens beim Einrast- bzw. Verankerungsvorgang mit der im Knochen befestigten Basiskomponente um einen definierten Betrag angehoben werden muß, um anschließend in die Verrastungen eingreifen zu können. Ein Aufweiten des Gelenkspaltes des Knies und eine daraus hervorgehende irreversible Dehnung des Bandapparates ist daher unvermeidlich und stellt vor allem bei einem an sich vor der endoprothetischen Versorgung vollkommen intakten Bandapparat ein zusätzliches OP-Trauma bei der Implantation der Knieendoprothese dar, das unter allen Umständen vermieden werden sollte.

Es ist daher Aufgabe der Erfindung, eine Tibiaplateaukomponente anzugeben, die eine minimalinvasive und einfache Implantation erlaubt und insbesondere eine irreversible Dehnung des Bandapparates vermeidet.

Die Aufgabe wird mit einer Tibiaplateaukomponente für eine Knieendoprothese gemäß den Merkmalen des Anspruchs 1 gelöst.

Die Tibiaplateaukomponente für eine Knieendoprothese umfasst eine Tibiagleitkomponente mit einer Tibiagleitfläche, die in gleitendem Kontakt mit einer Femurkomponente steht und eine in der Tibia verankerte, die Tibiagleitkomponente tragende Tibiabasiskomponente und ist erfindungsgemäß dadurch gekennzeichnet, dass die Tibiagleitfläche mindestens entlang eines Sagittalebenenschnittes die Form eines Zylindermantelausschnittes aufweist, wobei der Gleitflächenradius des Zylindermantelabschnittes gleich oder größer dem größten Radius der Femurkomponente ist. In Verbindung damit ist die Tibiaplateaukomponente weiterhin dadurch gekennzeichnet, dass die Kopplungsfläche zwischen der Tibiagleitkomponente und der Tibiabasiskomponente die Form eines Zylindermantelabschnittes hat und einen Kopplungsflächenradius aufweist, dessen Größe mindestens gleich der Größe des um den Betrag der mittleren Dicke der Tibiagleitkomponente erhöhten Gleitflächenradius ist.

Vorstehendes bedeutet, dass die mit der Femurkomponente in Kontakt stehende Gleitfläche, d.h. die Oberfläche der Tibiagleitkomponente, die Form eines Teils eines Zylindermantels aufweist. Die Unterseite der Tibiagleitkomponente wird ebenfalls durch die Form eines Zylindermantels beschrieben.

Der innere Radius des Zylindermantels entspricht erfindungsgemäß einem größten Radius der Femurkomponente. Die Größe des äußeren Radius des Zylindermantels wird durch Summe des inneren Radius zuzüglich der Dicke der Tibiagleitkomponente gebildet.

Weiterhin entspricht der äußere Radius des Zylindermantels dem Radius der Kopplungsfläche zwischen Tibiagleitkomponente und Tibiabasiskomponente. Bei der Implantation kann somit die Tibiagleitkomponente schubladenartig entlang des Radius der Kopplungsfläche der Tibiabasiskomponente in den Gelenkspalt zwischen Femur und Tibia hinein eingeführt werden. Dabei besteht zu keinem Zeitpunkt dieses Implantationsvorganges die Notwendigkeit, den Spalt zwischen Tibiabasiskomponente und Femurkomponente nennenswert aufzuweiten. Eine irreversible Dehnung des Bandapparates beim Einführen der Tibiagleitkomponente wird somit vermieden.

Zweckmäßigerweise sind die Tibiagleitkomponente und die Tibiabasiskomponente über eine an der Tibiagleitkomponente oder der Tibiabasiskomponente eingearbeitete Kopplungsnut in Verbindung mit einer an der jeweils anderen Komponente angeordneten Kopplungsbahn zusammengefügt. Eine solche zweckmäßige Ausgestaltung gewährleistet eine präzise Verbindung der Tibiagleitkomponente mit der Tibiabasiskomponente und sichert eine verschiebungssichere Lagerung von Tibiagleit- und -basiskomponente.

Weiterhin sind die Tibiagleitkomponente und die Tibiabasiskomponente miteinander gegen Relativbewegung verriegelt, insbesondere verrastet und/oder verschraubt. Dadurch wird eine stabile Lagerung der Tibiagleitkomponente auf der Tibiabasiskomponente erreicht.

Die Erfindung soll nachfolgend anhand der Figuren 1 bis 3c näher erläutert werden. Es werden für gleiche bzw. gleichartige Teile die selben Bezugszeichen verwendet.

### Hierbei zeigen:

- Fig. 1: eine beispielhafte Darstellung einer Femur- und Tibiakomponente in einem Sagittalebenenschnitt durch die Mitte einer Kondyle,
- Fig. 2: eine beispielhafte Darstellung einer Tibiagleitkomponente in Verbindung mit einer Tibiabasiskomponente in einer detaillierteren Ansicht,
- Fig. 3a-c: eine beispielhafte Darstellung eines Einbringvorganges der Tibiagleitkomponente in eine Anordnung aus Femur- und Tibiabasiskomponente in einem durch die Mitte einer Kondyle geführten Sagittalebenenschnitt;

- Fig. 4: eine perspektivische Darstellung der Tibiagleitkomponente und
- Fig. 5: eine perspektivische Darstellung der Tibiabasiskomponente.

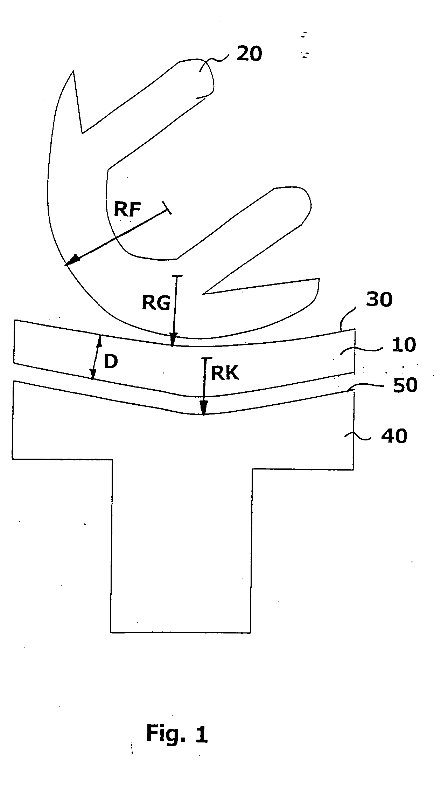

Fig. 1 stellt eine beispielhafte Ausführungsform einer erfindungsgemäßen Tibiaplateaukomponente dar. Die Tibiagleitkomponente 10 weist eine Tibiagleitfläche 30 auf, auf der die Femurkomponente 20 gleitet. Auf ihrer Unterseite ist die Tibiagleitkomponente auf der Tibiabasiskomponente 40 an einer Kopplungsfläche 50 befestigt. Die Tibiagleitfläche 30 und die Kopplungsfläche 50 weisen zueinander im wesentlichen konzentrische Zylindermantelflächen mit den Radien RG bzw. RK auf, die sich im wesentlichen um die Dicke D der Tibiagleitkomponente 10 unterscheiden. Die Ober- und Unterseite der Tibiagleitkomponente 10 bilden somit Abschnitte eines Zylindermantels.

Der Gleitflächenradius RG entspricht bei der in Fig. 1 dargestellten Ausführungsform einem größten Krümmungsradius RF der Femurkomponente 20. Da die Dicke der Tibiagleitkomponente 10 über die Länge in erster Näherung gleich bleibt, ist der sich aus der Summe aus Gleitflächenradius RG und Dicke D der Gleitkomponente ergebende Kopplungsradius RK gegenüber dem Gleitflächenradius um maximal 20% größer. Prinzipiell ist die Dicke D der Tibiagleitkomponente frei wählbar und kann an die jeweils vorliegenden anatomischen Verhältnisse des zu operierenden Kniegelenks angepaßt werden.

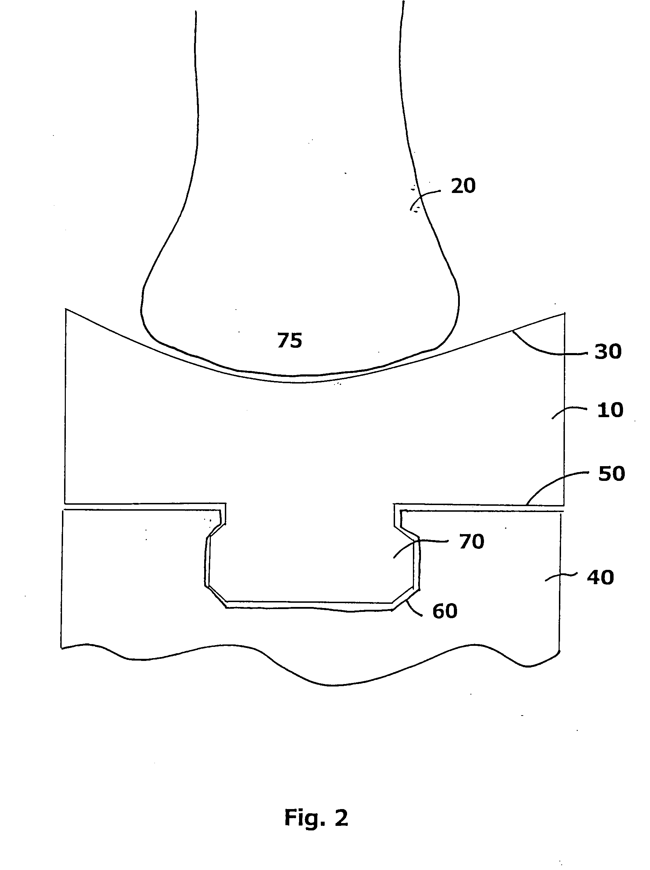

Fig. 2 zeigt einen Frontalebenenschnitt durch die Tibiagleitkomponente 10 und die Tibiabasiskomponente 40. Wie der Figur zu entnehmen ist, wird die Tibiagleitkomponente 10 durch das Zusammenwirken einer in dieser eingearbeiteten Kopplungsnut 60 und einer auf der Tibiabasiskomponente 40, insbesondere auf der Kopplungsfläche 50 angeordneten Kopplungsbahn 70 auf der Tibiabasiskomponente gegen laterale Verschiebungen bzw. gegen ein Abheben von der Tibiabasiskomponente gesichert. Die Kopplungsnut 60 bzw. die Kopplungsbahn 70 weisen zu diesem Zweck beispielsweise die in Fig. 2 gezeigte Trapez-Form auf.

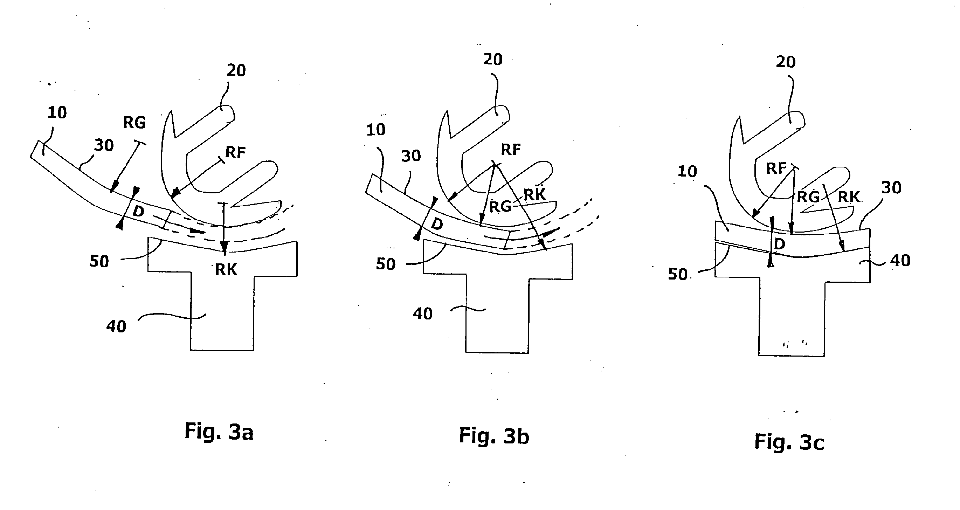

Die Zylindermantelform der Tibiagleitkomponente 10 und deren Vorteile werden in den Figuren 3a, 3b und 3c noch einmal verdeutlicht. Diese Figuren zeigen ein beispielhaftes gleitendes Einführen der Tibiagleitkomponente 10 entlang der Tibiagleitfläche 30 bzw. der Kopplungsfläche 50 bei einer bereits eingesetzten Femurkomponente 20 bzw. Tibiabasiskomponente 50. Beim Einschieben der Tibiagleitkomponente 10 folgt diesem dem Radius der Tibiabasiskomponente 40 und wird von der in Fig. 2 gezeigten Kopplungsnut 60 bzw. der Kopplungsbahn 70 geführt. Die Tibiagleitkomponente wird somit um die bereits implantierte Femurkomponente herum in das Knie eingebracht. Dabei vergrößert sich zu keinem Zeitpunkt nennenswert der Abstand zwischen Femur und Tibia und der Bandapparat wird somit nicht irreversibel gedehnt. Beginnend gemäß der Darstellung nach Fig. 3a und abschließend mit Fig. 3c ist die Tibiagleitkomponente 10 im Ansatz, zur Hälfte bzw. vollständig eingeschoben. Nachdem die Tibiagleitkomponente 10 vollständig eingeschoben worden ist, wird eine Verriegelung von Tibiabasiskomponente 40 und Tibiagleitkomponente 10 herbeigeführt. Diese Verriegelung erfolgt insbesondere durch eine Verschraubung oder Einrasten einer Schnappkontur, beispielsweise eines Stiftes.

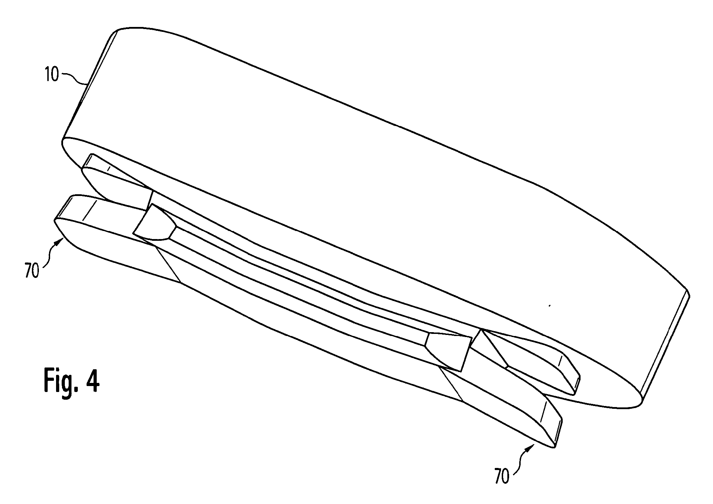

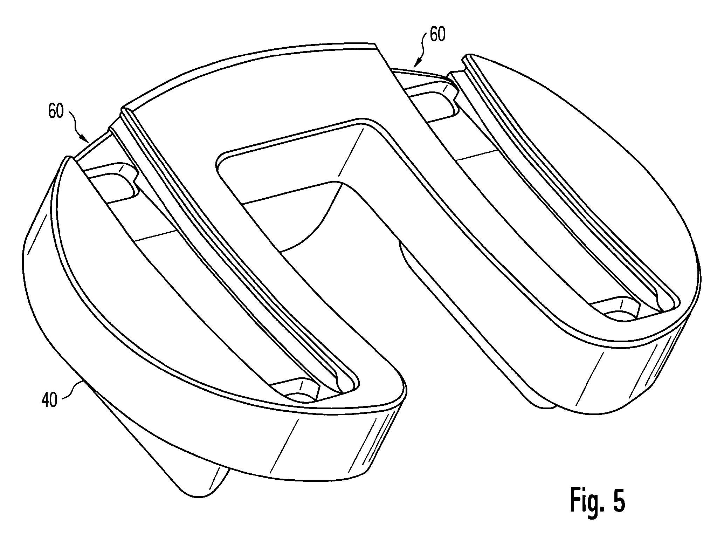

Fig. 4 zeigt eine perspektivische Darstellung der Tibiagleitkomponente und Fig. 5 eine perspektivische Darstellung der Tibiabasiskomponente.

Wie aus den Fig. 4 und 5 ersichtlich, ist die Kopplungsnut 60 paarig, und zwar entlang einer gedachten Symmetrieachse ausgeführt.
Analog ist die Kopplungsbahn 70 als komplementäres Teil, und zwar mit beispielsweise zungenartiger Gestalt ausgeführt. Die zungenartigen Fortsätze der Kopplungsbahn 70 können in die entsprechende Kopplungsnut eingeführt und dort arretiert werden. Die Verriegelung im eingeschobenen Zustand kann über eine stift- oder bolzenartige Befestigung, aber auch durch eine Snap-in-Verbindung oder dergleichen vorgenommen werden.

### Bezugszeichenliste

- 10: Tibiagleitkomponente
- 20: Femurkomponente
- 30: Tibiagleitfläche
- 40: Tibiabasiskomponente
- 50: Kopplungsfläche
- 60: Kopplungsnut
- 70: Kopplungsbahn
- 75: femurale Kondyle
- D: Dicke der Tibiagleitkomponente
- RF: größter Femurradius
- RG: Gleitflächenradius
- RK: Kopplungsflächenradius

## Patentansprüche

1. Tibiaplateaukomponente für eine Knieendoprothese, umfassend eine Tibiagleitkomponente (10) mit einer mit einer Femurkomponente (20) in gleitendem Kontakt stehenden Tibiagleitfläche (30) und eine in der Tibia verankerte, die Tibiagleitkomponente tragende Tibiabasiskomponente (40),
**dadurch gekennzeichnet, dass**
- die Tibiagleitfläche (30) mindestens entlang eines Sagittalebenenschnittes eine Form eines Zylindermantelausschnittes aufweist, wobei der Gleitflächenradius (RG) des Zylindermantelabschnittes gleich oder größer dem größten Femurradius (RF) der Femurkomponente (20) ist und
- eine Kopplungsfläche (50) zwischen der Tibiagleitkomponente (10) und der Tibiabasiskomponente (40) die Form eines Zylindermantelabschnittes hat und einen Kopplungsflächenradius (RK) aufweist, dessen Größe mindestens gleich der Größe des um den Betrag der mittleren Dicke (D) der Tibiagleitkomponente erhöhten Gleitflächenradius (RG) ist.

2. Tibiaplateaukomponente nach Anspruch 1,
**dadurch gekennzeichnet, dass**
die Tibiagleitkomponente (10) und die Tibiabasiskomponente (40) über eine an der Tibiagleitkomponente oder der Tibiabasiskomponente eingearbeitete Kopplungsnut (60) in Verbindung mit an einer der jeweils anderen Komponente befindlichen Kopplungsbahn (70) zusammengefügt sind.

3. Tibiaplateaukomponente nach einem der Ansprüche 1 oder 2,
**dadurch gekennzeichnet, dass**
die Tibiagleitkomponente (10) und die Tibiabasiskomponente (40) im verbundenen Zustand miteinander durch eine Schnappkontur verrastet und/oder verschraubt sind.
